# EUROPEAN PATENT SPECIFICATION

(11) **EP 3 274 101 B1**
(45) Date of publication and mention of the grant of the patent: **24.02.2021**
(21) Application number: 16711309.1
(22) Date of filing: 23.03.2016
(51) Int. Cl.: B05B 17/00, A61M 11/00, A61M 15/00, B05B 17/06

(54) **AN AEROSOL GENERATOR**
AEROSOLERZEUGER
GÉNÉRATEUR D'AÉROSOL

(30) Priority: 23.03.2015 EP 15160309
(43) Date of publication of application: 31.01.2018
(73) Proprietor: Stamford Devices Limited, Dangan, Galway (IE)
(72) Inventor: HYLAND, Kieran, County Kildare (IE); COYE, Colga, County Galway (IE); KEATING, Fran, County Galway (IE)
(74) Representative: Weldon O'Brien Ltd.
(86) International application number: PCT/EP2016/056413
(87) International publication number: WO 2016/151029

(56) References cited:
- WO-A1-2012/046220
- US-A1- 2002 129 812
- US-A1- 2003 192 959
- US-A1- 2005 077 376

## Description

### INTRODUCTION

### Field of the Invention

The invention relates to aerosol generators (or "nebulizers").

An example of an aerosol generator is described in WO2012/046220, and an illustration is provided in Fig. 1. In this aerosol generator (1) there is a liquid reservoir (2) delivering liquid onto an aperture plate or membrane (10). The membrane (10) is driven by an annular piezo element (11) and a washer (12), supported on a housing (15).

Such a nebulizer can aerosolise a broad range of liquids across a particle range of about 1 µm to 10µm mass median aerodynamic diameter (MMAD). The membrane (10) may be an electroformed plate with a plurality of holes, and is vibrated at a frequency of typically 127 to 157 kHz and this action breaks the surface tension of the liquid on the aperture plate and creates an aerosol plume as droplets pass through the aperture plate. Such systems are commonly called vibrating mesh technology.

The piezo element (11) is sensitive to liquid ingress and requires a method of sealing, and this is one of the functions achieved by using O-rings (13, 14). Alternatively the piezo may be sealed by being encapsulated in an elastomeric material. The size and geometry of the liquid reservoir exit is dictated by the inner diameter of the piezo element. The liquid reservoir exit opening may for example have a diameter greater than 4.5mm.

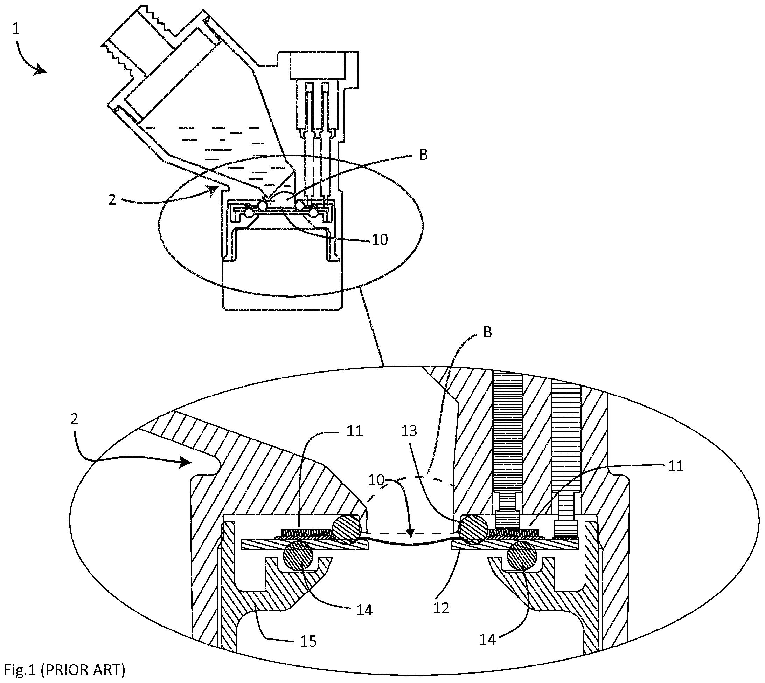

In use, an air bubble B can form at or near the liquid reservoir outlet on the entry side of the aperture plate as diagrammatically shown in Fig. 1.

A bubble may for example form during use if air becomes trapped between the membrane ("aperture plate") and liquid blocking the feed of liquid onto the membrane. This may be because the membrane in use pumps liquid from the reservoir but also pumps air back into the reservoir. An air bubble can also be formed through the introduction of medication when the membrane is not active. As the user administers the drug into the reservoir air can become trapped at the outlet of the reservoir. This is due to the narrow geometry of the reservoir outlet. The liquid enters as a wave, pressuring air in front until it is trapped between the membrane and the liquid, and again this air pocket blocks the feed of liquid onto the aperture plate.

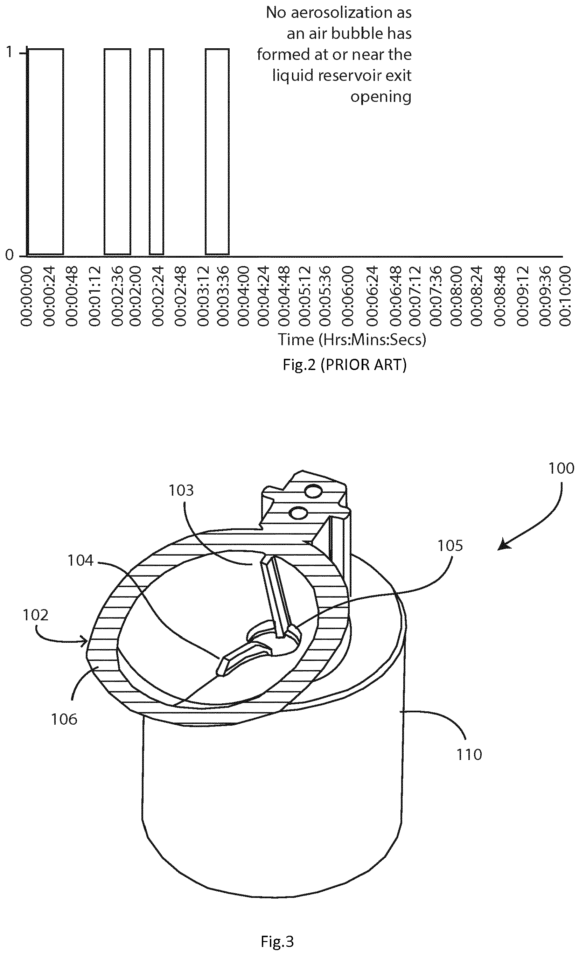

Fig. 2 depicts aerosolization plume on/off time, in which after approximately 4 minutes of treatment aerosolization ceases because an air bubble has formed at or near the liquid reservoir outlet.

Hess et al. US20050077376 and US2003/192959 discloses an air freshener comprising liquid reservoirs in the form of airless bags which supply liquid to be atomized into capillaries which in turn route the liquid to an internal space in a reciprocating movement between the two reservoirs. The internal space is between an actuator membrane and a nozzle below the membrane.

Litherland et al. US20020129812 discloses a liquid reservoir with a flow director which directs bubbles to migrate upwards into an outlet chamber separate from the liquid volume. It appears that provision of a separate outlet chamber for bubbles adds complexity.

The invention addresses this problem.

### SUMMARY OF THE INVENTION

According to the invention, there is provided a nebulizer reservoir as set out in claim 1.

Preferably, there are four vertices or sets of vertices equally spaced at relative angles of approximately 90°.

In a further aspect, the invention provides a method as set out in claim 3.

### Additional Statements

We also describe a nebulizer reservoir comprising a wall and a liquid outlet for engagement with a vibrating membrane assembly, the reservoir wall having an internal surface with at least one vertex for preventing formation of bubbles near the outlet.

In one example, which is not part of the present invention, the reservoir wall surface forms at least one groove or recess, the vertices being corners of said groove or recess.

In a further example, there is a plurality of vertices and said vertices are arranged around the reservoir at different circumferential positions.

### DETAILED DESCRIPTION OF THE INVENTION

### Brief Description of the Drawings

The invention will be more clearly understood from the following description of some embodiments thereof, given by way of example only with reference to the accompanying drawings in which:-
Fig. 1 is a diagrammatic side view of a nebulizer of the prior art, showing formation of a bubble, and Fig. 2 is a plot of operation if an air bubble forms;
Fig. 3 is a view from above, partly cut-away through the lower funnel portion of a reservoir of a nebulizer of the invention;
Fig. 4 is a diagram showing the configuration of a rib of the housing;
Fig. 5 is a perspective sketch showing use of the reservoir mounted at the inspiratory limb of a breathing circuit;
Fig. 6 is a perspective view showing mounting of the reservoir on the dry side of a humidifier;
Fig. 7 is a plot showing operation of the nebulizer of the invention, the vertical axis showing on-off levels and the horizontal axis showing time;
Fig. 8 is a perspective view showing geometry of a rib of a nebulizer of another embodiment; and
Fig. 9(a) to (h) is a set of schematic views showing in cross-section alternative configurations of bubble-prevention features in reservoir walls.

### Detailed Description of the Embodiments

A nebulizer liquid reservoir has an outlet for engagement with a vibrating membrane assembly to deliver liquid onto the aperture plate. In some embodiments, the outlet is at the throat area of a funnel-shaped part of the reservoir, through which the liquid is delivered onto the aperture plate.

The reservoir has at least one feature in its wall for preventing formation and/or retention of bubbles in the liquid near the outlet. Each reservoir wall feature has a vertex forming at least one corner. At least one feature is located near the reservoir outlet, and is elongate, and extends in a direction towards the outlet.

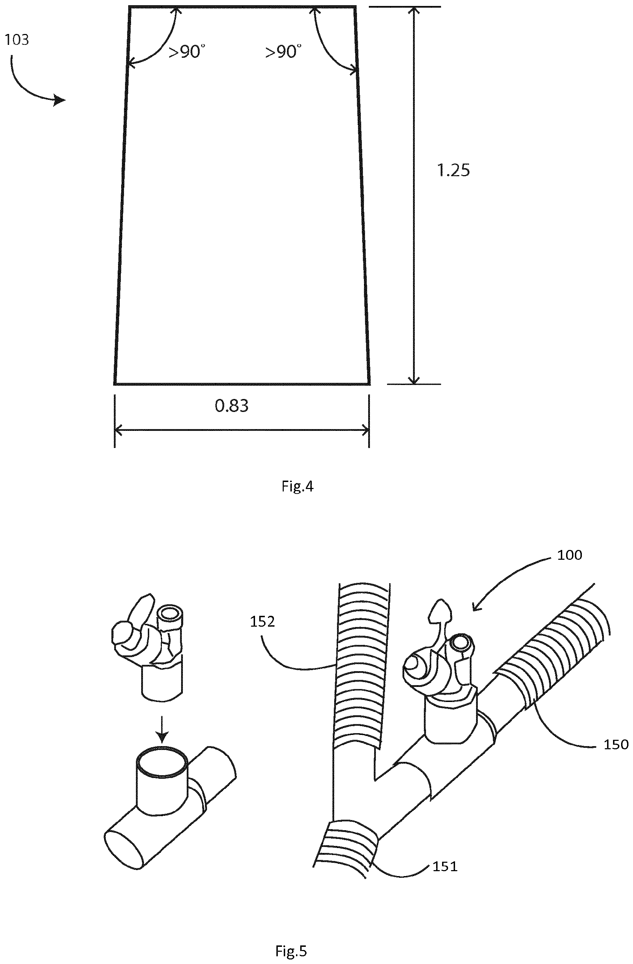

Referring to Figs. 3 to 5 a nebulizer 100 has a liquid supply reservoir with a funnel portion 102. The funnel portion 102 has bubble-prevention ribs 103 and 104 extending downwardly along a reservoir wall 106 towards a reservoir outlet 105. Configuration of the rib 103 is shown in Fig. 4, and that of the rib 104 is similar. In this case the rib depth is 1.25 mm, the maximum width is 0.83 mm, and the vertices or corners of the ribs have angles of just greater than 90°.

The outlet 105 is over an aperture plate or membrane at the top of an aerosol housing 110 in an arrangement generally similar to that of Fig. 1. The aerosol housing 110 includes the membrane, the drive piezo actuator, electrical connections, and aerosol outlet conduit, of conventional construction as shown in Fig. 1. The ribs 103 and 104 prevent formation of air bubbles between the aperture plate and (liquid) medication because any air will be expelled along the geometry formed by the rib and reservoir wall instead of being trapped and contributing to bubble formation. The ribs have vertices forming corners, and these are especially effective at allowing air to escape and so prevent air build-up.

As shown, the ribs 103 and 104 extend into the space of the reservoir outlet to an extent of 1.25 mm, and the two vertices (corners) are spaced by about 0.8 mm. The throat diameter is approximately 5mm, and so the total extent to which the ribs extend into the throat cross-section is about 50% in this example. It is preferred that the ribs extend to the maximum extent possible without occluding the access to the aperture plate. In general the bubble-prevention feature protrudes into the reservoir, the extent is in the range up to about 15% of the volume.

In use, the surface tension (typical range 38.93 to 70.57 mN/m (milliNewton per meter) of the liquid within the reservoir prevents an air-tight seal from forming along the length of the rib and at the reservoir outlet as the liquid does not create a seal against the surface of the medication reservoir i.e. a leak path is formed at the base of the rib 103 or 104. Any air that may build up either from the pressure at the reservoir outlet due to vibration of the aperture plate or through the introduction of medication into the reservoir is released along this leak path. This prevents an air bubble from forming at the reservoir outlet 105. It is envisaged that the ridge top corners prevent build-up of bubbles, and the inner corner where the rib adjoins the general reservoir internal surface provides an air escape route.

According to the invention, it is preferred that the bubble-preventing features comprise features such as ribs projecting into the volume of the reservoir, rather than recesses.

Referring to Fig. 5 the nebulizer 100 may be mounted on an inspiratory limb 150 near the Y-junction of a breathing circuit. This circuit also includes another limb 152 leading into the junction, and a common outlet limb 151. The reservoir is at an angle between horizontal and vertical when the limb 150 is horizontal. In this application the rib 104 is primarily in use, the rib 103 playing little or no role in bubble prevention.

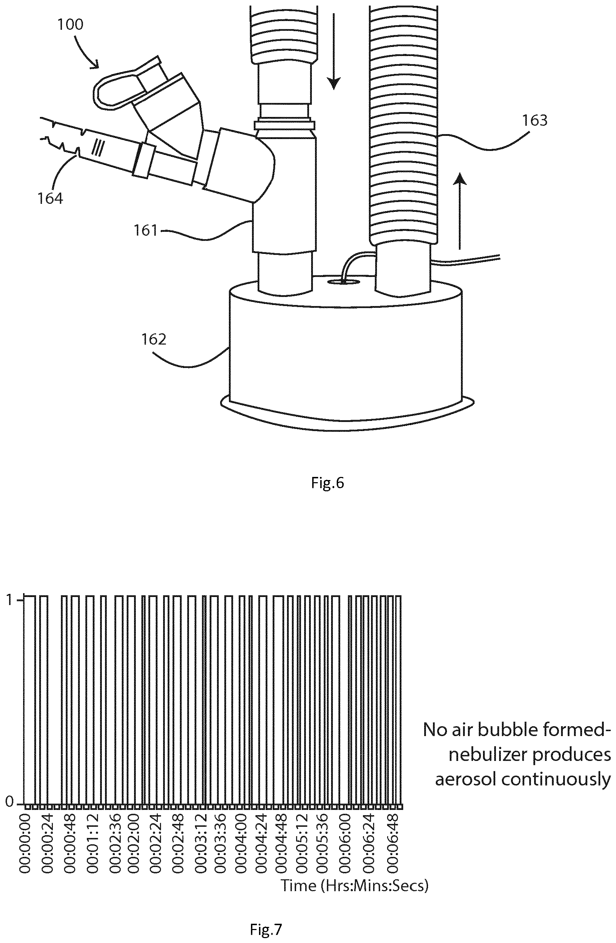

However, in another orientation the rib 103 plays the major role. An example is shown in Fig. 6, in which the nebulizer 100 is connected to a vertical limb 161 of a humidifier, near a T-piece 162 having an outflow limb 163. In this case there is a liquid fall along the side of the reservoir having the rib 103, and this rib performs the major bubble-prevention role. Fig. 6 shows an electrical connector 164 delivering power to the aperture plate.

It will be clear that with two opposed bubble-prevention features in the reservoir wall, the bubble-prevention advantage is obtained irrespective of which of the two recommended orientations are used. In many nebulizers there are two recommended orientations, and so two features is sufficient. However, if there is only one possible orientation, then one feature would be sufficient, or the feature on the lower side would be longer.

The reservoir extends at an angle to an axis of the aperture plate, according to the invention, the vertices are in a plane through the aperture plate central axis and the reservoir central axis. This plane is generally, in use, vertical, and so with opposed vertices on both sides in this plane there will typically be one feature at a side of the reservoir which is lowermost and which extends upwardly, in a vertical place. This is particularly effective for air escape to prevent bubble formation.

Fig. 7 depicts a predictable repeatable plume cycle of on off operation exhibited by this invention, as opposed to erratic and unpredictable long periods of no plume followed by variation in plume frequency.

The fact that the reservoir has only a single chamber allows efficient delivery of liquid onto the aperture plate, and also very effective bubble prevention. There is little ingress into the cross-sectional area of the throat leading to the aperture plate, but optimum bubble prevention.

It is envisaged that grooves or other recesses may be provided instead of ribs, to provide vertices. These may for example have a configuration which is an inverted form of the rib 103 shown in Fig. 4 However, these solutions are not part of the invention.

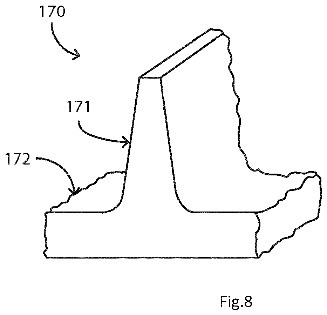

Fig. 8 shows another rib configuration 170, having side surfaces 171 extending from a reservoir wall internal surface 172. This configuration is particularly suitable for manufacture by a thermoplastic injection moulding process. An important consideration is that the rib does not cause visual or structural anomalies in the reservoir wall from which the rib protrudes. Typical anomalies would be sink marks and voids in the wall on the opposite side from the rib. These anomalies are caused by inefficient flow of the molten plastics material during the brief moulding cycle. Generous draft angles and the use of generous radii are preferred. This configuration allows manufacture in a sustainable repeatable manner to the correct quality and without blemish. The radius at the corner between the faces 171 and 172 is best kept as small as possible, creating a straw effect in a manner that allows air egress up along its length. However, a larger radius is preferable from a manufacturing perspective.

Regarding the throat, in general the larger the throat, the reduced likelihood of air-lock. The throat diameter in some embodiments is limited in by the piezo aperture plate drive geometry and the need to provide for a seal to protect the piezo from wetting. So in order to prevent the compounding effect of a small throat diameter and a round geometry favoured by bubbles, the ribs are introduced to disrupt this geometry and facilitate air egress.

In one embodiment the throat diameter is 4.5mm, giving a cross-sectional area of about 16 mm². The rib geometry reduces this area by 11%, to 14.15 mm².

In general, it is preferred to keep the rib as small as possible to minimise the impact on cross sectional area and to keep the geometry as sharp as possible to maximise the impact on airlock mitigation and droplet management

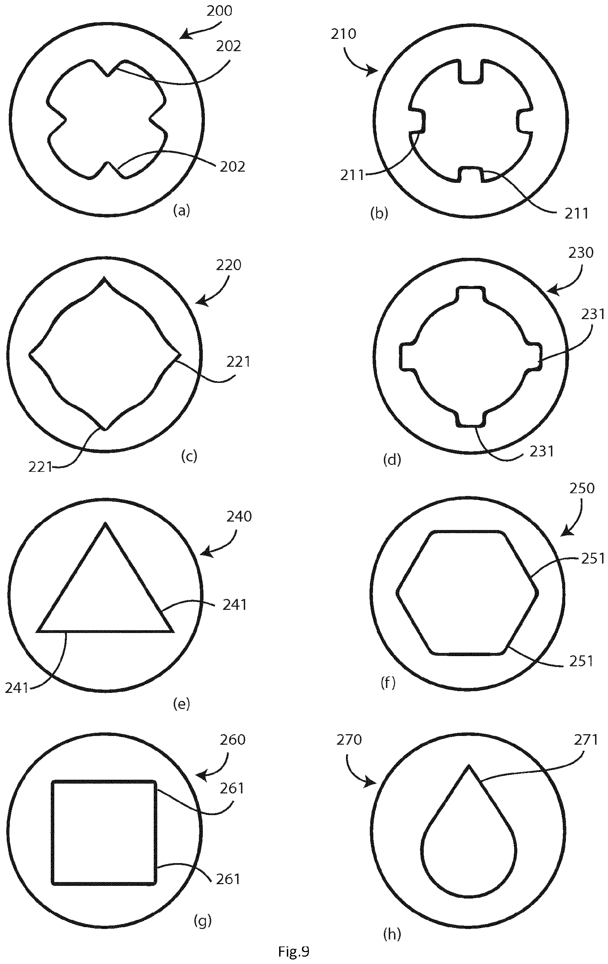

Figs. 9(a) to 9(h) are sketches showing cross-sectional views through reservoirs 200, 210, 220, 230, 240, 250, 260, and 270 respectively. Figs. 9(c) to 9(h) not falling within the scope of the appended claims. These all have vertices which extend in a direction towards the outlet (out of the plane of the page), which perform the air escape role as described above. In the reservoir 200 there are four triangular features 202 at 90° separations, each having a 90° angled corner facing into the centre of the reservoir.

In the reservoir 210 there are four rectangular ribs 211, also equally spaced around the reservoir wall. The reservoir 220 has four equally spaced triangular recesses 221 with corners facing radially outwardly. The reservoir 230 has four equally spaced rectangular recesses 231, as an inverse shape to the ridges 211. The reservoir 241 has a triangular cross-section, providing three corners 241 facing radially out. The reservoir 250 has an internal surface forming a hexagon, with six vertices 251 also facing radially out. The reservoir 260 has a square-shaped internal surface, providing four vertices 261 again facing radially out. The reservoir 270 has an internal surface configuration with only one internal vertex, 271.

The direction of the rib geometry longitudinally from the medication cup down to the aperture plate directs the medication down one side or another of the rib, thereby assisting the expulsion of air up the other side when the medication arrives into the vicinity of the throat area. The inclusion of two or more bubble-prevention features at different circumferential positions makes the device less orientation-dependent, ensuring that the anti-airlock feature is effective in either the vertical or horizontal orientation of the aperture plate. In any of the configurations of Fig. 9 there may be two opposed features rather than the number shown, especially if there are only two possible or recommended orientations in use.

Where the feature is a recess rather than a protrusion, as shown in some of the examples of Figs. 9(d) to (h) the recess on the opposing side to that of the pathway of the liquid acts as a straw type escape route for the air that is potentially trapped in the throat. In this embodiment the recess would be as small as achievable in the associated manufacturing process so as to allow movement of air before the liquid ingresses into the groove.

In examples, which are not part of the invention, where the feature includes a recess, this may be a variation on the aspect of features projecting into the reservoir space by being formed as the space between two or more adjacent ribs. Alternatively, it may be only an inverted form of a rib without any part protruding from the general reservoir wall surface. In this case it is envisaged that the corner with the general reservoir surface prevents bubble formation.

It will be appreciated that the ribbed feature(s) disrupt the circular geometry in the throat area of the reservoir. This disruption is significant as air bubbles favour round geometries. The ribbed feature(s) direct the medication down one side of the rib and upon arriving at the throat, facilitate escapement of the air up the other side. The ribs break the medication drop into smaller droplets of size less likely to hover over the air cushion and less likely therefore to occlude the throat.

Also, by providing at least two vertices at different circumferential positions around the reservoir the device is less sensitive to changes in orientation. The bubble-prevention aspect applies irrespective of the reservoir orientation.

It will also be appreciated that the invention is very simple, requiring only modifications to the wall shape of conventional reservoirs such as shown in Fig. 1. There is no need for multiple chambers, which could give rise to moulding difficulties and to less reliable operation.

The invention is not limited to the embodiments described but may be varied in construction and detail within the scope of the appended claims. As will be appreciated from Fig. 9 number and circumferential spacing of the vertices may vary. Also, they may or may not be equally spaced, for example there may be a concentration at locations where they would be more effective for an expected orientation of use.

## Claims

1. A nebulizer comprising a reservoir and a vibrating membrane assembly having a membrane (10) for receiving liquid from said reservoir, wherein the reservoir comprises a wall (106) and a liquid outlet (105) for engagement with the vibrating membrane assembly, wherein the reservoir wall (106) has an internal surface, the reservoir comprises a funnel-shaped portion (102) having said outlet (105) for delivering liquid onto the vibrating membrane,
the reservoir wall surface forms at least one bubble-prevention feature, said feature or features including a vertex of at least one rib (103, 104) protruding into the reservoir and having a corner forming said vertex, and said vertex is elongate and extends along the reservoir wall (106) towards the outlet (105), and the reservoir forms only a single chamber leading to the outlet (105), said reservoir extends at an acute angle to said membrane (10), the nebulizer has a use orientation, and said at least one vertex is located at a position where it is lowermost for said orientation,
wherein the reservoir extends at an acute angle to the outlet (105), in which an outlet central axis and a reservoir central axis are at said acute angle, wherein at least one vertex extends at or adjacent to a plane formed by said axes,
**characterized in that**,
there are at least two vertices including two opposed vertices substantially in said plane.

2. A nebulizer as claimed in the preceding claim, wherein there are four vertices or sets of vertices (202, 211, 221, 261) equally spaced at relative angles of approximately 90°.

3. A method of delivering liquid onto the nebulizer vibrating membrane (10) of the nebulizer of claim 1 or 2, the method comprising pouring the liquid into the reservoir of the nebulizer, said at least two vertices on the reservoir internal surface preventing build-up of air to a bubble stage, wherein the at least two vertices are located at a lowermost position of the reservoir, and wherein the at least two vertices are elongate and extend in a vertical plane towards the reservoir outlet (105).

## Patentansprüche

1. Vernebler, umfassend einen Behälter und eine Schwingmembrananordnung mit einer Membran (10) zum Aufnehmen von Flüssigkeit aus dem Behälter, wobei der Behälter eine Wand (106) und einen Flüssigkeitsauslass (105) zum Eingriff mit der Schwingmembrananordnung umfasst, wobei die Behälterwand (106) eine innere Oberfläche aufweist, der Behälter einen den Auslass (105) aufweisenden trichterförmigen Abschnitt (102) zum Zuführen von Flüssigkeit auf die Schwingmembran umfasst, die Behälterwandoberfläche mindestens ein Blasenverhinderungsmerkmal bildet, wobei das Merkmal oder die Merkmale einen Scheitel mindestens einer Rippe (103, 104), die in den Behälter ragt und eine den Scheitel bildende Ecke aufweist, umfassen und der Scheitel langgestreckt ist und sich entlang der Behälterwand (106) in Richtung des Auslasses (105) erstreckt, und der Behälter nur eine einzige Kammer bildet, die zu dem Auslass (105) führt, sich der Behälter unter einem spitzen Winkel zu der Membran (10) erstreckt, der Vernebler eine Gebrauchsorientierung aufweist und der mindestens eine Scheitel an einer Stelle liegt, wo er für die Orientierung am tiefsten ist, wobei sich der Behälter unter einem spitzen Winkel zu dem Auslass (105) erstreckt, wobei eine Auslassmittelachse und eine Behältermittelachse den spitzen Winkel bilden, wobei sich mindestens ein Scheitel an einer von den Achsen gebildeten Ebene oder dieser benachbart erstreckt, **dadurch gekennzeichnet, dass** es mindestens zwei Scheitel gibt, die zwei gegenüberliegende Scheitel im Wesentlichen in der Ebene umfassen.

2. Vernebler nach dem vorangehenden Anspruch, wobei es vier Scheitel oder Sätze von Scheiteln (202, 211, 221, 261) gibt, die in gleichmäßigen Abständen unter relativen Winkeln von ungefähr 90° angeordnet sind.

3. Verfahren zum Zuführen von Flüssigkeit auf die Verneblerschwingmembran (10) des Verneblers von Anspruch 1 oder 2, wobei das Verfahren das Gießen der Flüssigkeit in den Behälter des Verneblers umfasst, wobei die mindestens zwei Scheitel an der inneren Behälteroberfläche das Ansammeln von Luft zu einer Blasenstufe verhindern, wobei die mindestens zwei Scheitel an einer tiefsten Stelle des Behälters liegen, und wobei die mindestens zwei Scheitel langgestreckt sind und sich in einer vertikalen Ebene in Richtung des Behälterauslasses (105) erstrecken.

## Revendications

1. Nébuliseur comprenant un réservoir et un ensemble de membrane vibrante ayant une membrane (10) pour recevoir du liquide dudit réservoir, dans lequel le réservoir comprend une paroi (106) et une sortie de liquide (105) pour un engagement avec l'ensemble de membrane vibrante, dans lequel la paroi de réservoir (106) a une surface interne,
le réservoir comprend une partie en forme d'entonnoir (102) ayant ladite sortie (105) pour distribuer du liquide sur la membrane vibrante,
la surface de paroi de réservoir forme au moins une caractéristique de prévention de bulles, ladite caractéristique ou lesdites caractéristiques incluant un sommet d'au moins une nervure (103, 104) faisant saillie dans le réservoir et ayant un coin formant ledit sommet, et ledit sommet est allongé et s'étend le long de la paroi de réservoir (106) vers la sortie (105), et
le réservoir ne forme qu'une seule chambre menant à la sortie (105),
ledit réservoir s'étend selon un angle aigu par rapport à ladite membrane (10),
le nébuliseur a une orientation d'utilisation, et ledit au moins un sommet est situé à une position où il est le plus bas pour ladite orientation,
dans lequel le réservoir s'étend selon un angle aigu par rapport à la sortie (105), dans lequel un axe central de sortie et un axe central de réservoir se trouvent selon ledit angle aigu, dans lequel au moins un sommet s'étend au niveau d'un plan ou adjacent à un plan formé par lesdits axes,
**caractérisé en ce que**,
il y a au moins deux sommets incluant deux sommets opposés sensiblement dans ledit plan.

2. Nébuliseur tel que revendiqué dans la revendication précédente, dans lequel il y a quatre sommets ou ensembles de sommets (202, 211, 221, 261) également espacés selon des angles relatifs d'approximativement 90°.

3. Procédé de distribution de liquide sur la membrane vibrante de nébuliseur (10) du nébuliseur selon la revendication 1 ou 2, le procédé comprenant le versement du liquide dans le réservoir du nébuliseur, lesdits au moins deux sommets sur la surface interne de réservoir empêchant l'accumulation d'air à un stade de bulle, dans lequel les au moins deux sommets sont situés à une position la plus basse du réservoir, et dans lequel les au moins deux sommets sont allongés et s'étendent dans un plan vertical vers la sortie de réservoir (105).
